# EUROPEAN PATENT APPLICATION

(11) **EP 1 247 570 A2**
(43) Date of publication of application: **09.10.2002**
(21) Application number: 02252348.4
(22) Date of filing: 28.03.2002
(51) Int. Cl.: B01J 19/00, G06F 19/00, C07H 21/04

(54) **Chemical array reading**

(30) Priority: 30.03.2001 US 280525 P; 31.07.2001 US 919555
(71) Applicant: Agilent Technologies, Inc., Palo Alto, CA 94306 (US)
(72) Inventor: Cattell, Herbert F., Mountain View, CA 94040 (US); Delenstarr, Glenda C., Belmont, CA 94002 (US)
(74) Representative: Jehan, Robert

(57) **Abstract**

A method apparatus, and computer program products for generating and using an addressable array of chemical moieties on a substrate. In a processing unit may retrieve array related data from a memory. Such array related data may comprise any of data on a characteristic of the fabricated array, an instruction for reading an array, or an instruction on processing data from a read array. The processing unit may automatically access a program routine for reading the array or processing data from the read array based on the retrieved data.

## Description

This invention relates to arrays, particularly biopolymer arrays (such polynucleotide arrays, and particularly DNA arrays) which are useful in diagnostic, screening, gene expression analysis, and other applications.

Arrays of biopolymers, such as arrays of peptides or polynucleotides (such as DNA or RNA), are known and are used, for example, as diagnostic or screening tools. Such arrays include regions (sometimes referenced as features or spots) of usually different sequence biopolymers arranged in a predetermined configuration on a substrate. The arrays, when exposed to a sample, will exhibit a pattern of binding which is indicative of the presence and/or concentration of one or more components of the sample, such as an antigen in the case of a peptide array or a polynucleotide of particular sequence in the case of a polynucleotide array. The binding pattern can be detected by interrogating the array, for example, by observing a fluorescence pattern on the array following exposure to a fluid sample in which all potential targets (for example, DNA) in the sample have been labeled with a suitable fluorescent label.

Biopolymer arrays can be fabricated using either in situ synthesis methods or deposition of the previously obtained biopolymers. The in situ synthesis methods include those described in US 5,449,754 for synthesizing peptide arrays, as well as WO 98/41531 and the references cited therein for synthesizing polynucleotides (specifically, DNA). Such in situ synthesis methods can be basically regarded as iterating the sequence of depositing drops of: (a) a protected monomer onto predetermined locations on a substrate to link with either a suitably activated substrate surface (or with a previously deposited deprotected monomer); (b) deprotecting the deposited monomer so that it can now react with a subsequently deposited protected monomer; and (c) depositing another protected monomer for linking. Different monomers may be deposited at different regions on the substrate during any one iteration so that the different regions of the completed array will have different desired biopolymer sequences. One or more intermediate further steps may be required in each iteration, such as oxidation and washing steps.

The "deposition method" basically involve depositing previously obtained biopolymers at predetermined locations on a substrate which are suitably activated such that the biopolymers can link thereto. The deposited biopolymers may, for example, be obtained from synthetic or biological sources. Biopolymers of different sequence may be deposited at different regions of the substrate to yield the completed array. Washing or other additional steps may also be used. Typical procedures known in the art for deposition of polynucleotides, particularly DNA such as whole oligomers or cDNA, are to load a small volume of DNA in solution in one or more drop dispensers such as the tip of a pin or in an open capillary and, touch the pin or capillary to the surface of the substrate. Such a procedure is described in US 5,807,522. When the fluid touches the surface, some of the fluid is transferred. The pin or capillary must be washed prior to picking up the next type of DNA for spotting onto the array. This process is repeated for many different sequences and, eventually, the desired array is formed. Alternatively, the DNA can be loaded into a drop dispenser in the form of an inkjet head and fired onto the substrate. Such a technique has been described, for example, in PCT publications WO 95/25116 and WO 98/41531, and elsewhere. This method has the advantage of non-contact deposition. Still other methods include pipetting and positive displacement pumps such as the Biodot equipment (available from Bio-Dot Inc., Irvine CA, USA).

In array fabrication, the quantities of DNA available for the array are usually very small and expensive. Sample quantities available for testing are usually also very small and it is therefore desirable to simultaneously test the same sample against a large number of different probes on an array. These conditions require use of arrays with large numbers of very small, closely spaced spots. Due to the precision required, and to maintain costs low, it will often be desirable to have the arrays fabricated at a fabrication facility and then shipped to the end user. As mentioned above, the end user will typically expose the received array to a labeled sample and read the array to obtain data on the binding pattern of the sample on the array. However, due to the small size of array features and depending on the strength to which a target sequence may bind to a feature, the detected signal from any given feature as a result of binding of a target to be detected may be small and may include significant "noise". Such noise may include binding of other than target sequences (non-target sequences) for a feature, to that feature. Additionally, there may be a general non-specific background binding signal which should be subtracted from the total signal detected at a feature. As well, there may be errors in one or more features introduced during the array fabrication process (for example, one or more features is not present or of an incorrect size) or on a particular type of array a certain type of feature (for example, a class of control probes) is not present. Various computer implemented program routines may be used for processing the read data and accounting for noise using statistical methods or suspected or known array feature errors (such as can be visibly seen on a screen displaying an image of the detected signal data). However, it would be desirable to provide a simple means by which such routines can detect array characteristics which may affect how a program routine is applied (including whether it should be applied at all) and respond accordingly.

The present invention seeks to provide improved chemical arrays. Aspects of the present invention are specified in claims 1, 13 and 28.

The present invention provides in one aspect, a method of producing an addressable array of chemical moieties, such as different sequence biopolymers (for example, polynucleotides such as DNA) on a substrate, as might be executed by a fabrication apparatus. The method includes depositing the biopolymers onto different regions of the substrate so as to fabricate the array. Array related data is saved in a memory, and this may be done in association with an identifier (for example, a unique identifier). Such array related data may include any of (that is, any one or more of) data on a characteristic of the fabricated array, an instruction for reading an array, or an instruction on processing data from a read array.

The identifier may be applied to the substrate or a housing carrying the substrate. The fabricated array with applied map identifier may then be shipped to a remote location or optionally used locally.

The method may additionally include retrieving the array related data from the memory and communicating the identity map to a remote location (for example, in response to receiving a communication of the identifier from the remote location). In an alternative embodiment, the memory may comprise a portable storage medium which is shipped to a remote location (for example, to the same location to which the fabricated array is shipped).

Optionally, the method may also include applying a communication address to the substrate or a housing carrying the substrate. Such communication address identifies a location from which the identity map will be communicated in response to a received communication of the associated map identifier. The communication address may, for example be a network address such as an address on a public phone network, a Local Area Network (LAN), or a Wide Area Network (WAN). For example, the communication address may be a network address of a database at the fabrication site, which database contains multiple identity maps and associated map identifiers, which correspond to array fabrication orders from different remote customers.

The method may in one aspect be executed at a central fabrication station which receives array fabrication orders from multiple remote customers. One or more arrays may be fabricated for each such customer according to a method of the present invention. Each fabricated array and identifier may be shipped to the same location from which the corresponding biopolymer set and customer order were received.

Another aspect of the present invention provides a method of using an addressable array of biopolymers on a substrate. The array may be received by the user (or, a user may simply receive read array data and an array identifier, for example). The following steps are then executed (for example, in a processing unit). Array related data is retrieved from a memory, which array related data may comprise any of data on a characteristic of the fabricated array, an instruction for reading an array, or an instruction on processing data from a read array. A program routine for reading the array or processing data from the read array is automatically accessed based on the retrieved data.

In the method of using, the array may be received with an a associated identifier (for example, the previously mentioned identifier applied to the array substrate or housing). In this case, the method may additionally include reading the identifier (for example, machine reading the identifier), and wherein the array related data is retrieved based on the identifier. Thus, reading of the array or processing information obtained from reading of the array, may be controlled (in whole or in part) in accordance with the retrieved array related data.

The accessing of the program routine based on the array related data may be done in a number of ways and for a number of purposes. In one example, the processing unit automatically presents the user (for example, after reading of the array but before processing of any read results) with an opportunity for making one or more possible selections or alerts the user as to a selection. The presenting of the foregoing opportunity can also be done in a number of ways. For example, the processing unit may generate and display a list of possible selections for the user based on the retrieved array related data. In another example, the program routine may normally present the user with an opportunity for making one or more selections, however the processing unit prevents the user from making a particular selection (that is, the processing unit simply refuses to accept that particular selection). The selections may be simultaneously displayed. Even a particular selection which the user is prevented from making (sometimes referenced as an "invalid selection") by the processing unit based on the retrieved array related data, may still be displayed although it cannot be selected. In this situation, the invalid selection may be presented in a different color from valid selections (that is, those selections which are allowed). The selections may be of different routines for reading the array or processing data from a read array.

In the case where the memory is a remote database, the identifier may be communicated to the remote database and the array related data received in response. Alternatively, where the memory is a portable storage medium received from the fabrication station, the array related data map may be obtained from that storage medium using the identifier (and a suitable reader, such as a disk drive in the case where the portable storage medium is a magnetic or optical disk). One way of determining a communication address of a remote location to which the map identifier should be communicated, is by machine reading the communication address from the substrate or the housing.

The present invention also provides an apparatus for producing an addressable array of biopolymers on a substrate, which apparatus can execute one or more steps of any one or more methods of producing an array. In one aspect, the apparatus includes an array fabricator to deposit the biopolymers onto different regions of the substrate so as to fabricate the array. Such apparatus further includes a processor to save in a memory the array related data in association with an identifier. The apparatus may include a writing system which applies the identifier to the substrate or a housing carrying the substrate. Optionally, the processor may perform other functions of any one or more of the methods described above, and the apparatus may include a memory and/or a writer for the computer readable storage medium described above. Such an apparatus may, for example, be used as a centralized array fabrication station to fabricate custom arrays from many different remote customers in accordance with instructions received from them.

The present invention further provides an apparatus for receiving an addressable array of biopolymers on a substrate, which apparatus can execute one or more steps of any of the methods of using the array as described above. In one embodiment, this apparatus may include a reader which reads the identifier carried on the array substrate or a housing for the array substrate. A processor retrieves array related data based on the identifier. This may be obtained, for example, by communicating the map identifier to a remote location and receiving the identity map in response, or by retrieving the identity map from a portable storage medium using the map identifier. Optionally, the same or a different reader of the apparatus may be used to read the communication address on the substrate or the housing and the processor communicates the map identifier to the read address. Any apparatus of the foregoing type may be part of an array reader which reads the addressable array of biopolymers on a substrate (for example, after exposure of the array to a sample). Such an array reader additionally includes a holder to receive the array or a housing carrying the array, and a sensor to read signals from respective features on the array.

Apparatus and methods of the present invention, can be also be used to deposit drops of any other fluid moiety or moieties, and embodiments of the apparatus can be described by replacing "biopolymer", "polynucleotide", or similar terms with "chemical moiety" or "moiety". Furthermore, some components of the present invention may be used independently of the others. For example, the identifier applied to the array or housing can be used to retrieve from a remote or other location, in any of the described manners described herein, any information relating to the corresponding array (such as array layout information) which has been saved in a memory in association with that that identifier.

There is further provided by the present invention a computer program product, comprising: a computer readable storage medium having a computer program stored thereon for performing, when loaded into a computer, can execute any of the methods of any one or more methods of the present invention.

One or more of the various aspects of the present invention may provide one or more of the following, or other, useful benefits. For example, a user is provided with a simple way of reading an array or processing data from a read array, based on characteristics of that array.

Embodiments of the invention will now be described with reference to the drawings in which:
FIG. 1 illustrates a substrate carrying multiple arrays, such as may be fabricated by methods of the present invention;
FIG. 2 is an enlarged view of a portion of FIG. 1 showing multiple ideal spots or features;
FIG. 3 is an enlarged illustration of a portion of the substrate in FIG. 2;
FIG. 4 illustrates a screen displayed to a user during a preferred method of the present invention;
FIG. 5 is a schematic diagram of a preferred fabrication apparatus and method according to the present invention; and
FIG. 6 is a schematic diagram of an apparatus at a user site which can execute a preferred method according to the present invention.

In the present application, unless a contrary intention appears, the following terms refer to the indicated characteristics. A "biopolymer" is a polymer of one or more types of repeating units. Biopolymers are typically found in biological systems and particularly include polysaccharides (such as carbohydrates), and peptides (which term is used to include polypeptides and proteins) and polynucleotides as well as their analogs such as those compounds composed of or containing amino acid analogs or non-amino acid groups, or nucleotide analogs or non-nucleotide groups. This includes polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in Watson-Crick type hydrogen bonding interactions. Polynucleotides include single or multiple stranded configurations, where one or more of the strands may or may not be completely aligned with another. A "nucleotide" refers to a sub-unit of a nucleic acid and has a phosphate group, a 5 carbon sugar and a nitrogen containing base, as well as functional analogs (whether synthetic or naturally occurring) of such sub-units which in the polymer form (as a polynucleotide) can hybridize with naturally occurring polynucleotides in a sequence specific manner analogous to that of two naturally occurring polynucleotides.. For example, a "biopolymer" includes DNA (including cDNA), RNA, oligonucleotides, and PNA and other polynucleotides as described in US 5,948,902 and references cited therein (all of which are incorporated herein by reference), regardless of the source. An "oligonucleotide" generally refers to a nucleotide multimer of about 10 to 100 nucleotides in length, while a "polynucleotide" includes a nucleotide multimer having any number of nucleotides. A "biomonomer" references a single unit, which can be linked with the same or other biomonomers to form a biopolymer (for example, a single amino acid or nucleotide with two linking groups one or both of which may have removable protecting groups). A biomonomer fluid or biopolymer fluid reference a liquid containing either a biomonomer or biopolymer, respectively (typically in solution).

An "array", unless a contrary intention appears, includes any one or two dimensional arrangement of addressable regions bearing a particular chemical moiety to moieties (for example, biopolymers such as polynucleotide sequences) associated with that region. An array is "addressable" in that it has multiple regions of different moieties (for example, different polynucleotide sequences) such that a region (a "feature" or "spot" of the array) at a particular predetermined location (an "address") on the array will detect a particular target or class of targets (although a feature may incidentally detect non-targets of that feature). Array features are typically, but need not be, separated by intervening spaces. In the case of an array, the "target" will be referenced as a moiety in a mobile phase (typically fluid), to be detected by probes ("target probes") which are bound to the substrate at the various regions. However, either of the "target" or "target probes" may be the one which is to be evaluated by the other (thus, either one could be an unknown mixture of polynucleotides to be evaluated by binding with the other). An "array layout" refers collectively to one or more characteristics of the features, such as feature positioning, one or more feature dimensions, errors, or some indication of a moiety at a given location (for example, a biopolymer sequence), or whether a given type of feature is present (for example, a particular type of control feature). "Hybridizing" and "binding", with respect to polynucleotides, are used interchangeably.

When one item is indicated as being "remote" from another, this is referenced that the two items are at least in different buildings, and may be at least one mile, ten miles, or at least one hundred miles apart. "Communicating" information references transmitting the data representing that information as electrical signals over a suitable communication channel (for example, a private or public network). "Forwarding" an item refers to any means of getting that item from one location to the next, whether by physically transporting that item (such as by shipping) and includes, at least in the case of data, physically transporting a medium carrying the data or communicating the data. An array "package" may be the array plus only a substrate on which the array is deposited, although the package may include other features (such as a housing with a chamber). A "chamber" references an enclosed volume (although a chamber may be accessible through one or more ports). It will also be appreciated that throughout the present application, that words such as "top", "upper", and "lower" are used in a relative sense only. "Fluid" is used herein to reference a liquid. A "set" or a "sub-set" may have one or more members (for example, one or more drops).

A "pulse jet" is any device which can dispense drops in the formation of an array. Pulse jets operate by delivering a pulse of pressure (such as by a piezoelectric or thermoelectric element) to liquid adjacent an outlet or orifice such that a drop will be dispensed therefrom. A "processor" or "processing unit" references any combination of hardware or software which can control components as required to execute recited steps and includes, for example, a general purpose digital microprocessor suitably programmed (for example, from a computer readable medium carrying necessary program code or by communication from a remote location) to perform all of the steps required of it, or any hardware or software combination which will perform those or equivalent steps. Reference to a singular item, includes the possibility that there are plural of the same items present. The use of the word "may" in the present application indicates that something is optional. Steps recited in any method herein, may be carried out in the recited order or in any other order that is logically possible. All patents and other cited references are incorporated into this application by reference except insofar as where any definitions in those references conflict with those of the present application (in which case the definitions of the present application are to prevail).

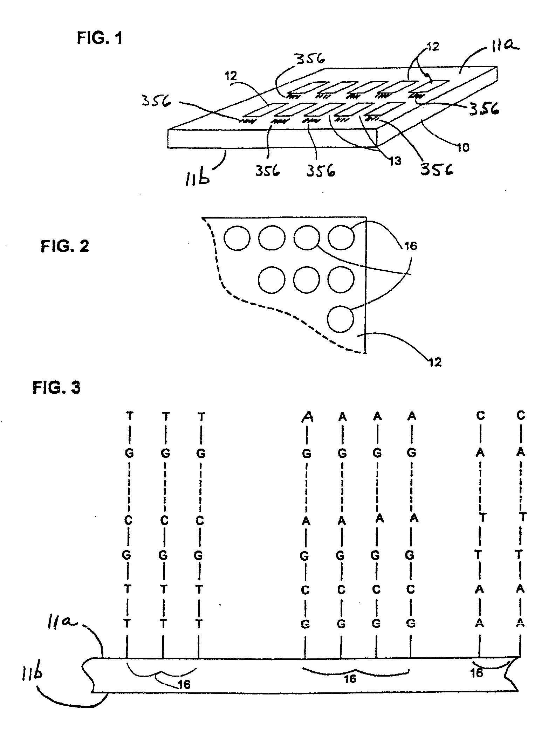

Referring first to FIGS. 1-3, typically embodiments of methods and apparatus invention generate or use a contiguous planar substrate 10 carrying one or more arrays 12 disposed across a front surface 11a of substrate 10 and separated by inter-array areas 13. A back side 11b of substrate 10 does not carry any arrays 12. The arrays on substrate 10 can be designed for testing against any type of sample, whether a trial sample, reference sample, a combination of them, or a known mixture of polynucleotides (in which latter case the arrays may be composed of features carrying unknown sequences to be evaluated). Each array 12 has associated with it a unique identifier in the form of a bar code 356 described below. By "unique" in this sense does not mean the identifier is absolutely unique, but it is sufficiently long so as unlikely to be confused with another identifier on another array (and is preferably unique as to a particular fabrication station on a given communication channel). While ten arrays 12 are shown in FIG. 1 and the different embodiments described below may use a substrate with only one array 12 on it, it will be understood that substrate 10 and the embodiments to be used with it may have any number of desired arrays 12. Similarly, substrate 10 may be of any shape, and any apparatus used with it adapted accordingly. Depending upon intended use, any or all of arrays 12 may be the same or different from one another and each will contain multiple spots or features 16 of biopolymers such as polynucleotides. A typical array may contain from more than ten, more than one hundred, more than one thousand or ten thousand features, or even more than from one hundred thousand features. All of the features 16 may be different, or some or all could be the same. In the embodiment illustrated, there are interfeature areas 17 between features, which do not carry any polynucleotide. It will be appreciated though, that the interfeature areas 17 could be of various sizes and configurations. It will be appreciated that there need not be any space separating arrays 12 from one another, nor features 16 within an array from one another. However, in the case where arrays 12 are formed by the deposition method as described above, such inter-array and inter-feature areas 17 will typically be present. Each feature carries a predetermined polynucleotide (which includes the possibility of mixtures of polynucleotides). As per usual, A, C, G, T represent the usual nucleotides. It will be understood that there may be a linker molecule (not shown) of any known types between the front surface 11a and the first nucleotide.

FIGS. 2 and 3 are enlarged views illustrating portions of ideal features where the actual features formed are the same as the desired features (sometimes referenced as the "target" or "aim" features), with each feature 16 being uniform in shape, size and composition, and the features being regularly spaced. In practice, such an ideal result is difficult to obtain.

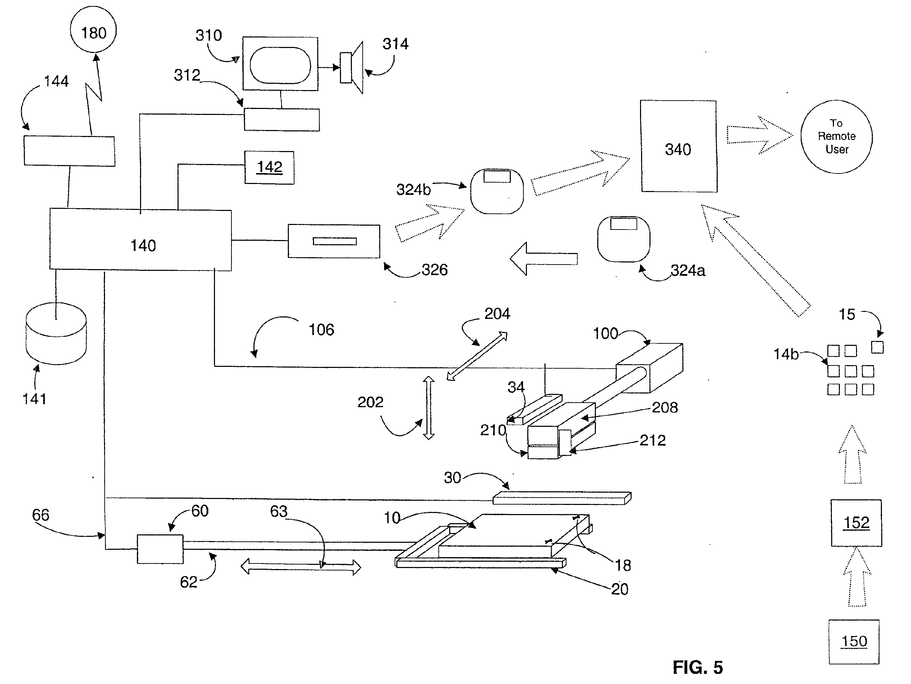

Referring now to FIG. 5, preferred apparatus which can execute the methods disclosed herein will now be described. The apparatus of FIG. 5 is a fabrication station which includes a substrate station 20 on which can be mounted a substrate 10. Pins or similar means (not shown) can be provided on substrate station 20 by which to approximately align substrate 10 to a nominal position thereon. Substrate station 20 can include a vacuum chuck connected to a suitable vacuum source (not shown) to retain a substrate 10 without exerting too much pressure thereon, since substrate 14 is often made of glass.

A dispensing head 210 is retained by a head retainer 208. The positioning system includes a carriage 62 connected to a first transporter 60 controlled by processor 140 through line 66, and a second transporter 100 controlled by processor 140 through line 106. Transporter 60 and carriage 62 are used execute one axis positioning of station 20 (and hence mounted substrate 10) facing the dispensing head 210, by moving it in the direction of arrow 63, while transporter 100 is used to provide adjustment of the position of head retainer 208 (and hence head 210) in a direction of axis 204. In this manner, head 210 can be scanned line by line, by scanning along a line over substrate 10 in the direction of axis 204 using transporter 100, while line by line movement of substrate 10 in a direction of axis 63 is provided by transporter 60. Transporter 60 can also move a load station (not shown) beneath head 210 such that polynucleotides or other biopolymers obtained from different vessels from a customer, can be loaded into head 210. Such a load station and method of use is described in detail in U.S. Patent Application Serial No. 09/183,604 for "Method And Apparatus For Liquid Transfer" filed Oct. 30, 1998 by Tella et al, incorporated herein by reference. Head 210 may also optionally be moved in a vertical direction 202, by another suitable transporter (not shown). It will be appreciated that other scanning configurations could be used. It will also be appreciated that both transporters 60 and 100, or either one of them, with suitable construction, could be used to perform the foregoing scanning of head 210 with respect to substrate 10. Thus, when the present application recites "moving" or "positioning" one element (such as head 210) in relation to another element (such as one of the stations 20 or substrate 10) it will be understood that any required moving can be accomplished by moving either element or a combination of both of them. The head 210, the positioning system, and processor 140 together act as the deposition system of the apparatus. An encoder 30 communicates with processor 140 to provide data on the exact location of substrate station 20 (and hence substrate 10 if positioned correctly on substrate station 20), while encoder 34 provides data on the exact location of holder 208 (and hence head 210 if positioned correctly on holder 208). Any suitable encoder, such as an optical encoder, may be used which provides data on linear position.

Processor 140 also has access through a communication module 144 to a communication channel 180 to communicate with a remote station. Communication channel 180 may, for example, be a Wide Area Network ("WAN"), telephone network, satellite network, or any other suitable communication channel. Communication module 144 may be any module suitable for the type of communication channel used, such as a computer network card, a computer fax card or machine, or a telephone or satellite modem. A reader 142 further communicates with processor 140. Reader 142 is capable of reading the identity of multiple vessels received from a customer (for example, reading the well identifiers such as those described above). Where the trays 360, 364, 368 carry only an identifier in the form of a reference mark 372 or the like, processor 140 is programmed to assign individual vessel identifiers based on such reference mark 372 as read by reader 142. The biopolymers for placing in the load station for subsequent loading into head 210, can be obtained from the vessels (such as wells 362) using an automated or manual procedure, or combination of the foregoing. Such "obtaining" procedure may include purification, amplification, reverse transcription, or the like, as mentioned above.

Head 210 may have multiple pulse jets, such as piezoelectric or thermoelectric type pulse jets as may be commonly used in an ink jet type of printer and may, for example, include multiple chambers each communicating with a corresponding set of multiple drop dispensing orifices and multiple ejectors which are positioned in the chambers opposite respective orifices. Each ejector is in the form of an electrical resistor operating as a heating element under control of processor 140 (although piezoelectric elements could be used instead). Each orifice with its associated ejector and portion of the chamber, defines a corresponding pulse jet. It will be appreciated that head 210 could, for example, have more or less pulse jets as desired (for example, at least ten or at least one hundred pulse jets). Application of a single electric pulse to an ejector will cause a drop to be dispensed from a corresponding orifice. Certain elements of the head 210 can be adapted from parts of a commercially available thermal inkjet print head device available from Hewlett-Packard Co. as part no. HP51645A. A suitable head construction is described in U.S. Patent Application Serial No. 09/150,507 filed Sept. 9, 1998 by Caren et al. for "Method And Multiple Reservoir Apparatus For Fabrication Of Biomolecular Arrays", Alternatively, multiple heads could be used instead of a single head 210, each being similar in construction to head 210 and being movable in unison by the same transporter or being provided with respective transporters under control of processor 140 for independent movement.

As is well known in the ink jet print art, the amount of fluid that is expelled in a single activation event of a pulse jet, can be controlled by changing one or more of a number of parameters, including the orifice diameter, the orifice length (thickness of the orifice member at the orifice), the size of the deposition chamber, and the size of the heating element, among others. The amount of fluid that is expelled during a single activation event is generally in the range about 0.1 to 1000 pL, usually about 0.5 to 500 pL and more usually about 1.0 to 250 pL. A typical velocity at which the fluid is expelled from the chamber is more than about 1 m/s, usually more than about 10 m/s, and may be as great as about 20 m/s or greater. As will be appreciated, if the orifice is in motion with respect to the receiving surface at the time an ejector is activated, the actual site of deposition of the material will not be the location that is at the moment of activation in a line-of-sight relation to the orifice, but will be a location that is predictable for the given distances and velocities.

The apparatus can deposit drops to provide features which may have widths (that is, diameter, for a round spot) in the range from a minimum of about 10 µm to a maximum of about 1.0 cm. In embodiments where very small spot sizes or feature sizes are desired, material can be deposited according to the invention in small spots whose width is in the range about 1.0 µm to 1.0 mm, usually about 5.0 µm to 500 µm, and more usually about 10 µm to 200 µm.

The apparatus further includes a monitor 310, speaker 314, and operator input device 312. Operator input device 312 may, for example, be a keyboard, mouse, or the like. Processor 140 has access to a memory 141, and controls print head 210 (specifically, the activation of the ejectors therein), operation of the positioning system, operation of each jet in print head 210, and operation of monitor 310 and speaker 314. Memory 141 may be any suitable device in which processor 140 can store and retrieve data, such as magnetic, optical, or solid state storage devices (including magnetic or optical disks or tape or RAM, or any other suitable device, either fixed or portable). Processor 140 may include a general purpose digital microprocessor suitably programmed from a computer readable medium carrying necessary program code, to execute all of the steps required for by the present invention for array production, or any hardware or software combination which will perform those or equivalent steps. The programming can be provided remotely to processor 141, or previously saved in a computer program product such as memory 141 or some other portable or fixed computer readable storage medium using any of those devices mentioned below in connection with memory 141. For example, a magnetic or optical disk 324a may carry the programming, and can be read by disk writer/reader 326.

A writing system which is under the control of processor 140, includes a writer in the form of a printer 150 which apply identifiers onto substrate 10 by printing them in the form of the bar codes 356 directly onto substrate 10 (or indirectly such as onto a label later attached to the substrate), each in association with a corresponding array 12 as illustrated in FIG. 1. Alternatively, the identifiers can by applied onto a housing carrying the substrate or label to be applied to such substrate or housing. Printer 150 may accomplish this task before or after formation of the array by the drop deposition system. The identifiers may include the map identifier and may also optionally include a communication address which identifies the address of a remote location on communication channel 180 from which the identity map will be communicated in response to a received communication of the associated map identifier. Such remote location may be that of communication module 144 or alternatively that of another accessible memory on a communication channel carrying the database of identity maps and associated map identifiers. Examples of a communication address may be a telephone number, computer ID on a WAN, or an internet Universal Resource Locator. The writing system further includes a data writer/reader 326 (such as an optical or magnetic disk drive) which can write data to a portable computer readable storage medium (such as an optical or magnetic disk). A cutter 152 is provided to cut substrate 10 into individual array units 15 each carrying a corresponding array 12 and bar code 356.

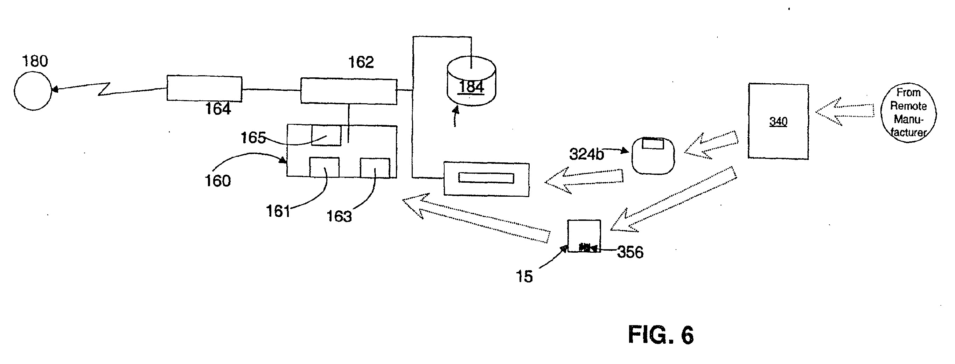

The above described components in FIG. 5 represent an apparatus for producing an addressable array, which is sometimes referenced herein as a "fabrication station". FIG. 6 illustrates an apparatus for receiving an addressable array 12, in particular a single "user station", which is remote from the fabrication station (usually at the location of the customer which ordered the received array 12). The user station includes a processor 162, a memory 184, a scanner 160 which can read an array, data writer/reader 186 (which may be capable of writing/reading to the same type of media as writer/reader 320), and a communication module 164 which also has access to communication channel 180. Scanner 160 may include a holder 161 which receives and holds an array unit 15, as well as a source of illumination (such as a laser) and a light sensor 165 to read fluorescent light signals from respective features on the array. Communication module 164 may be any type of suitable communication module, such as those described in connection with communication module 144. Memory 184 can be any type of memory such as those used for memory 141. Scanner 160 can be any suitable apparatus for reading an array, such as one which can read the location and intensity of fluorescence at each feature of an array following exposure to a fluorescently labeled sample. For example, such a scanner may be similar to the GENEARRAY scanner available from Hewlett-Packard, Palo Alto, CA. Scanner 160 also includes though, a reader 163 to read a bar code 356 appearing on segment 15. The scanning components of scanner 160, holder 161, and reader 163 may all be contained within the same housing of a single same apparatus.

It will be understood that there may be multiple such user stations, each remote from the fabrication station and each other, in which case the fabrication station acts as a central fabrication station (that is, a fabrication station which services more than one remote user station at the same or different times). One or more such user stations may be in communication with the fabrication station at any given time. It will also be appreciated that processors 140 and 162 can be programmed from any computer readable medium carrying a suitable computer program. For example, such a medium can be any memory device such as those described in connection with memory 141, and may be read locally (such as by reader/writer 320 in the case of processor 140 or writer/reader 186 in the case of processor 162) or from a remote location through communication channel 180.

The operation of the fabrication station will now be described. It will be assumed that a substrate 10 on which arrays 12 are to be fabricated, is in position on station 20 and that processor 140 is programmed with the necessary layout information to fabricate target arrays 12. Processor 140 controls fabrication of an array 12 for each biopolymer set received in multiple vessels, by depositing one or more drops of each biopolymer of the set onto a corresponding region (feature) on the substrate so as to fabricate the array in the manner described above. During fabrication, any errors in deposited drops or features can be detected by a camera 212 which views the drops immediately following deposition. A suitable arrangement and process is described in detail in U.S. Patent Applications: "Polynucleotide Array Fabrication", Serial No. 09/302898 filed April 30/99 by Caren et al.; and "Biopolymer Array Inspection", Serial No. 09/419447 filed Oct. 15/99 by Fisher.

Array related data may be stored in memory 141 in association with the corresponding identifier for the same array 12. Alternatively or additionally, the array related data and associated identifier for one or more arrays 12 which are to be shipped to a same customer, can be stored onto a portable storage medium 324b by writer/reader 326 for provision to the remote customer. Such array related data may include data on any characteristic of the fabricated array such as array layout information, feature error or other characteristic information, as well as a machine (such as a computer) readable instruction on reading of the array, or an instruction on processing data from a read array.

Either before array fabrication on substrate 10 has been commenced, or after it has been completed, substrate 10 may be sent to writer 150 which, under control of processor 140, writes the identifier for each array 12 in the form of bar codes 356 onto substrate 10 each in association with its corresponding array (by being physically close to it in the manner shown in FIG. 1). The substrate 10 is then sent to a cutter 152 wherein portions of substrate 10 carrying an individual array 12 and its associated identifier 356 are separated from the remainder of substrate 10, to provide multiple array units 15. The array unit 15 is placed in package 340 along with storage medium 324b (if used) carrying at least the identity map and map identifier for that same array unit 15 (and possibly for other array units 15 which are to be sent to the same remote customer location), and the package then shipped to a remote user station.

The above sequence can be repeated at the fabrication station as desired for multiple substrates 10 in turn. As mentioned above, the fabrication station may act as a central fabrication station for each of multiple remote user stations, in the same manner as described above. Whether or not the fabrication station acts as a central fabrication station, it can optionally maintain a database of unique map identifiers in memory 141, each in association with the corresponding identity map.

At the user station of FIG. 6, the resulting package 340 is then received from the remote fabrication station. A sample, for example a test sample, is exposed to the array 12 on the array unit 15 received in package 340. Following hybridization and washing in a known manner, the array unit 15 is then inserted into holder 161 in scanner 160 and read by it to obtain read results (such as information representing the fluorescence pattern on the array 12). The reader 163 in scanner 160 also reads the identifier 356 present on the array substrate 10 in association with the corresponding array 12, while the array unit 15 is still positioned in retained in holder 161. Using identifier 356, processor 162 may then retrieve the corresponding array related data for array 12 from portable storage medium 324b or from the database of such information in memory 141 by communicating the associated identifier to that database through communication module 164 and communication channel 180 and receiving the corresponding identity map in response. In the latter situation, processor 162 may obtain the communication address of communication module 144 by which to access memory 141 (or the address of another database carrying the identity map and associated identifier of array 12), from the communication address in identifier 356.

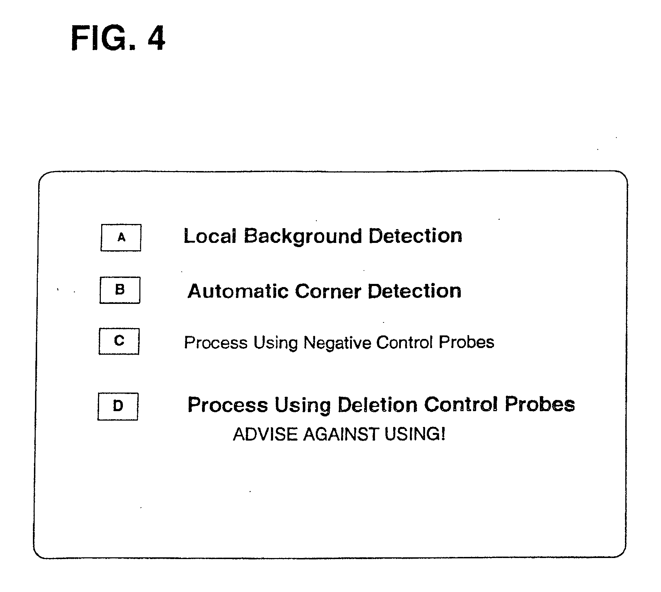

Once processor 162 has retrieved the array related data it can use such data to either control reading of the array or to process information obtained from reading the array. This can be done by processor 162 accessing a program routine normally used for reading the array or processing data from the read array based on the retrieved array related data. For example, the processor 162 may present the user with an opportunity for making one or more possible selections or alert the user to a selection (for example is warn against, or suggest against or in favor of), based on the retrieved array related data. One method of doing this is illustrated in FIG. 4. FIG. 4 is a screen which may be displayed on monitor 310. In FIG. 4 multiple possible selections of different algorithms for reading the array or processing read data from the array are shown. Selections A and B represent local background detection and automatic corner detection algorithms, as disclosed in U.S. Patent Application "Method And System For Extracting Data From Surface Array Deposited Features" Serial No. 09/659415 filed Sept. 11, 2000 by Enderwick et al. Selections C and D represent algorithms for subtracting total detected signal from a feature due to non-target binding, by the use of data read from array features 16 containing negative control probe or deletion control probes, as described in U.S. Patent Applications Serial No. 09/350969 for "Methods For Controlling Cross-Hybridization In Analysis Of Nucleic Acid Sequences" filed July 9, 1999, and Serial No. 09/398399 for "Techniques For Assessing Nonspecific Binding Of Nucleic Acids To Surfaces" filed Sept. 17, 1999.

A user can normally select any of the read data processing algorithms by clicking on box A through C. However, as illustrated in FIG. 4, processor 162 has retrieved array related data which indicates that there are no negative control probes on the array. Thus, selection C is still displayed (for example, in a different color or font) but cannot be selected by a user. Similarly, the processing using deletion control probes may still be selected by a user, but the retrieved array related data indicates that one or more deletion control probe carrying features may have errors which may make their use unreliable. Thus, the user is alerted against using the algorithm which relies on deletion control probes by the phrase "ADVISE AGAINST USING!". However, selections which are invalid may simply not be displayed at all, and processor 162 may simply generate and display a list of valid selections from the retrieved array related data. Alternatively, if the retrieved array related data indicates that only a certain feature or features have an error (for example, only one negative control probe feature), processor 162 may access the program routine by allowing a user to continue to select selection C but simply instruct that routine not to use the data from those features in error.

The array related data may be constructed of two segments if desired. One segment may have data for all instances of a particular array layout design, while the other segment has data relating to a specific array of that design with which the array related data is associated. Default parameters may be established in the design file, which may be over-ridden by specific array file. For example, a negative control probe can be generally indicated in the design file, but for one or more particular arrays this may be over-ridden at processor 162 by the array specific file if the associated array does not contain any negative control probes. Similarly, a particular negative control probe feature which is in error can be instructed by the array specific file to be ignored at processor 162 although the design file would indicate that negative control probe algorithm selections are available. If both negative and deletion control probes were available, algorithms using either one could be selected by processor 162 according to the design file. However, if there were errors in one of those types of control probes on a specific array, the array specific file could instruct processor 162 to over-ride the normal allowance of a selection of algorithms based on the error type probes provided in a design file.

As already mentioned, results from the array reading can be processed results, such as obtained by rejecting a reading for a feature which is below a predetermined threshold and/or forming conclusions based on the pattern read from the array (such as whether or not a particular target sequence may have been present in the sample). The results of the reading (processed or not) can be forwarded (such as by communication) to be received at a remote location for further evaluation and/or processing, or use, using communication channel 180 or reader/writer 186 and medium 190. This data may be transmitted by others as required to reach the remote location, or re-transmitted to elsewhere as desired.

In a variation of the above, it is possible that each array 12 and its substrate 10 may be contained with a suitable housing. Such a housing may include a closed chamber accessible through one or more ports normally closed by septa, which carries the substrate 10. In this case, the identifier for each array may be applied to the housing.

Modifications in the particular embodiments described above are, of course, possible. For example, where a pattern of arrays is desired, any of a variety of geometries may be constructed other than the organized rows and columns of arrays 12 of FIG. 1. For example, arrays 12 can be arranged in a series of curvilinear rows across the substrate surface (for example, a series of concentric circles or semi-circles of spots), and the like. Similarly, the pattern of regions 16 may be varied from the organized rows and columns of spots in FIG. 2 to include; for example, a series of curvilinear rows across the substrate surface(for example, a series of concentric circles or semi-circles of spots), and the like. While the features 16 are normally laid out in regular arrangements (such as rows and columns), even irregular arrangements of the arrays or the regions within them can be used provided a means is available to the end user to know the identity and location of features (such as being provided in the array related data, specifically the array layout information contained therein).

The present methods and apparatus may be used to deposit biopolymers or other moieties on surfaces of any of a variety of different substrates, including both flexible and rigid substrates. Preferred materials provide physical support for the deposited material and endure the conditions of the deposition process and of any subsequent treatment or handling or processing that may be encountered in the use of the particular array. The array substrate may take any of a variety of configurations ranging from simple to complex. Thus, the substrate could have generally planar form, as for example a slide or plate configuration, such as a rectangular or square or disc. In many embodiments, the substrate will be shaped generally as a rectangular solid, having a length in the range about 4 mm to 200 mm, usually about 4 mm to 150 mm, more usually about 4 mm to 125 mm; a width in the range about 4 mm to 200 mm, usually about 4 mm to 120 mm and more usually about 4 mm to 80 mm; and a thickness in the range about 0.01 mm to 5.0 mm, usually from about 0.1 mm to 2 mm and more usually from about 0.2 to 1 mm. However, larger substrates can be used, particularly when such are cut after fabrication into smaller size substrates carrying a smaller total number of arrays 12. Substrates of other configurations and equivalent areas can be chosen. The configuration of the array may be selected according to manufacturing, handling, and use considerations.

The substrates may be fabricated from any of a variety of materials. In certain embodiments, such as for example where production of binding pair arrays for use in research and related applications is desired, the materials from which the substrate may be fabricated should ideally exhibit a low level of non-specific binding during hybridization events. In many situations, it will also be preferable to employ a material that is transparent to visible and/or UV light. For flexible substrates, materials of interest include: nylon, both modified and unmodified, nitrocellulose, polypropylene, and the like, where a nylon membrane, as well as derivatives thereof, may be particularly useful in this embodiment. For rigid substrates, specific materials of interest include: glass; fused silica, silicon, plastics (for example, polytetrafluoroethylene, polypropylene, polystyrene, polycarbonate, and blends thereof, and the like); metals (for example, gold, platinum, and the like).

The substrate surface onto which the polynucleotide compositions or other moieties is deposited may be porous or non-porous, smooth or substantially planar, or have irregularities, such as depressions or elevations. The surface may be modified with one or more different layers of compounds that serve to modify the properties of the surface in a desirable manner. Such modification layers, when present, will generally range in thickness from a monomolecular thickness to about 1 mm, usually from a monomolecular thickness to about 0.1 mm and more usually from a monomolecular thickness to about 0.001 mm. Modification layers of interest include: inorganic and organic layers such as metals, metal oxides, polymers, small organic molecules and the like. Polymeric layers of interest include layers of: peptides, proteins, polynucleic acids or mimetics thereof (for example, peptide nucleic acids and the like); polysaccharides, phospholipids, polyurethanes, polyesters, polycarbonates, polyureas, polyamides, polyethyleneamines, polyarylene sulfides, polysiloxanes, polyimides, polyacetates, and the like, where the polymers may be hetero- or homopolymeric, and may or may not have separate functional moieties attached thereto (for example, conjugated),

The disclosures in United States patent applications no. 60/280,525 and no. 09/919,555, from which this application claims priority, and in the abstract accompanying this application are incorporated by reference.

## Claims

1. A method of generating an addressable array of chemical moieties on a substrate, including the steps of:
(a) depositing the moieties onto different regions of the substrate so as to fabricate the array;
(b) saving in a memory array related data, which array related data may comprise any of data on a characteristic of the fabricated array, an instruction for reading an array, or an instruction on processing data from a read array;
(c) shipping the fabricated array, and forwarding the array related data to a remote location; and
(d) applying a communication address to the substrate or a housing carrying the substrate, which communication address identifies a remote location from which the array related data will be communicated in response to a received communication of the associated identifier.

2. A method according to claim 1, including the steps of:
saving the array related data in association with an identifier;
associating the identifier with the array; and
shipping the fabricated array, and forwarding the identifier to a remote location.

3. A method according to claim 2 wherein the identifiier is associated with the array by applying the identifier to the substrate or a housing carrying the substrate.

4. A method according to claim 3 wherein the chemical moieites are biopolymers.

5. A method according to claim 4 wherein the biopolymers are DNA.

6. A method according to claim 3 wherein the memory is a database, the method additionally comprising retrieving the array related datafrom the memory and communicating the retrieved data to a remote location in response to receiving a communication of the identifier from the remote location.

7. A method according to claim 3 wherein the memory comprises a portable storage medium, the method additionally comprising shipping the portable storage medium to a remote location.

8. A method according to claim 7 wherein the portable storage medium is shipped to the same remote location as the array.

9. A method according to claim 3, wherein the method is performred for multiple arrays at a central fabrication station, and wherein each of the fabricated arrays with applied identifier is shipped to one or more of the remote stations.

10. A method according to claim 9 wherein the chemical moieties are polynucleotides.

11. A method according to claim 9wherein the polynucleotides are DNA.

12. A method according to claim 9 including the step of applying a same communication address to each of the substrates or housings carrying the substrates, which communication address identifies a remote location from which each array related data map will be communicated in response to a received communication of the associated identifier.

13. A method of using an addressable array of chemical moieties on a substrate, including the steps of:
(a) receiving the addressable array;and
(b) in a processing unit:
(i) retrieving array related data from a memory, which array related data may comprise any of data on a characteristic of the fabricated array, an instruction for reading an array, or an instruction on processing data from a read array; and
(ii) automatically accessing a program routine for reading the array based on the retrieved data.

14. A method according to claim 13 wherein the array is received with an associated identifier and the method additionally comprises reading the identifier, and wherein the program routine is accessed based on the identifier.

15. A method according to claim 14 wherein the identifier is carried on a substrate for the array, or a housing carrying the substrate.

16. A method according to claim 14 including the step of retrieving array related data based on the identifier, and wherein the processing unit automatically presents the user with an opportunity for making one or more possible selections or alerts the user as to a selection based on the retrieved array related data.

17. A method according to claim 16 wherein the processing unit automatically presents the user with an opportunity for making one or more possible selections based on the retrieved array related data by displaying a list of possible selections for a user.

18. A method according to claim 16 wherein the program routine normally presents the user with an opportunity for making one or more possible selections, and wherein the processing unit automatically alerts the user as to a selection, or prevents the user from making a particular selection, based on the retrieved array related data.

19. A method according to claim 16 wherein the program routine normally presents the user with an opportunity for making multiple selections by simultaneously displaying the multiple selections, and wherein the particular selection which the user is prevented from making a based on the retrieved array related data is still displayed.

20. A method according to claim 16 wherein the selections are of different routines for reading the array or processing data from a read array.

21. A method according to claim 14 wherein the memory is a remote database, the method additionally comprising communicating the read identifier to the remote database and receiving in response the array related data.

22. A method according to claim 14 wherein the memory is a portable storage medium received from a remote location.

23. A method according to claim 15 including the steps of:
machine reading a communication address on the substrate or the housing; and
communicating the identifier to the communication address and receiving the associated array related data in response.

24. A method according to claim 15 including the steps of:
exposing the array to a sample; and
reading the array following the exposure to the sample.

25. A method according to claim 24 wherein the array is read in a same apparatus in which the identifier is read.

26. A method comprising forwarding a result of an array reading obtained by a method of claim 24, to a remote location.

27. A method comprising transmitting or receiving data representing a result of an array reading obtained by a method of claim 24.

28. An apparatus for receiving an addressable array of biopolymers on a substrate, including a processor operable to:
(i) retrieve array related data from a memory, which array related data may comprise any of data on a characteristic of the fabricated array, an instruction for reading an array, or an instruction on processing data from a read array; and
ii) automatically access a program routine for reading the array or processing data from the read array based on the retrieved data.

29. An apparatus according to claim 28 including a reader operable to read an identifier carried on an array substrate or a housing for the array, and wherein the processor is operable to retrieve array related data based on the read identifier.

30. An apparatus according to claim 29 wherein the processor can communicate the read identifier to a remote location and to receive the array related data in response.

31. An apparatus according to claim 30 wherein the reader is operable to read a communication address on the substrate or the housing, and wherein the processor can communicate the identifier to the read address.
